# EUROPEAN PATENT APPLICATION

(11) **EP 1 350 515 A1**
(43) Date of publication of application: **08.10.2003**
(21) Application number: 02252469.8
(22) Date of filing: 05.04.2002
(51) Int. Cl.: A61K 33/14, A61P 9/12

(54) **Low-dose potassium supplementation for the prevention and treatment of hypertension**

(71) Applicant: BTG INTERNATIONAL LIMITED, London EC4M 7SB (GB)
(72) Inventor: The designation of the inventor has not yet been filed
(74) Representative: Dolan, Anthony Patrick

(57) **Abstract**

The present invention provides methods of decreasing blood pressure in a mammal, and treating or preventing hypertension in a mammal by administering to the mammal between about 10 mmol/day and about 35 mmol/day of a potassium salt. The present invention also provides supplemented beverages and foodstuffs which provide, as a daily serving, a supplement of a potassium salt between about 10 mmol/day and about 35 mmol/day.

## Description

### FIELD OF THE INVENTION

The present invention is directed, in part, to methods of decreasing blood pressure, treating or preventing hypertension, and to supplemented beverages and foodstuffs comprising low doses of potassium for carrying out the methods.

### BACKGROUND OF THE INVENTION

Hypertension is an increasingly common chronic condition of people living in the most industrialized countries, and is associated with an increased risk of cardiovascular and cerebrovascular disease (Whelton, *Lancet,* 1994, 344, 101-6). The association between vascular diseases and blood pressure (BP) appears to be present even among non-hypertensive subjects with normal to high BP (Kannel, *J. Amer. Med. Assoc*., 1996, 275, 1571-6). A small decrease in BP may lead to a significant decrease in the incidence and prevalence of hypertension in the population (Hypertension Prevention Trial Research Group, *Arch. Intern. Med.,* 1990, 150, 153-62), reducing considerably mortality and morbidity of high-BP related diseases (Whelton, *Lancet,* 1994, 344, 101-6). High blood pressure is thus an important modifiable risk factor for vascular diseases.

The non-pharmacological management of BP is an important and widely accepted means of preventing and treating hypertension (Joint National Committee on Detection, Evaluation, and Treatment of High Blood Pressure, *Arch. Intern. Med.,* 1997, 157, 2413-46). Amongst the factors influencing BP much attention has been paid to differences in diet. Intra-population studies have shown that not only are vegetarian diets consistently found to be associated with low BP (Sacks *et al*., *Am. J. Clin. Nutr.,* 1988, 48 (3 Suppl), 795-800) and to be effective in lowering BP in both normotensive and hypertensive people (Rouse *et al., Lancet,* 1983, 1, 5-10) but that an increased intake of fruits and vegetables may have a significant hypotensive effect (Appel *et al., N. Engl. J. Med.,* 1997, 336, 1117-24), focusing attention on nutrients derived largely from these foods.

Attention is currently focused on dietary sodium, and particularly on the sodium-potassium ratio as factors influencing BP (Dyer *et al., Hypertension,* 1994, 23, 729-36). Although sodium restriction is widely recommended as a non-pharmacological means of preventing and treating hypertension (Joint National Committee on Detection, Evaluation, and Treatment of High Blood Pressure, *Arch. Intern. Med.,* 1997, 157, 2413-46) the epidemiological and clinical studies that have advocated this approach are intensely debated (Freedman *et al., Eval. Rev*., 2001, 25, 267-87). The decrease in sodium consumption necessary to exert a small decrease in blood pressure (100 mmol/day) (Dyer *et al., Hypertension,* 1994, 23, 729-36) is acknowledged to be difficult to achieve and to sustain (*Sinaiko* et al., Hypertension, *1993, 21, 989-94),* and might even represent a risk for some in the population (*Graudal* et al., J. Amer. Med. Assoc., *1998, 279, 1383-91).* Moreover sodium restriction might be beneficial only for a fraction of the population defined as salt-sensitive (Morris *et al., Hypertension*, 1999, 33, 18-23). A strong interconnection between the intake of the two electrolytes and BP level is suggested by observational studies, since the urinary sodium-potassium ratio seems to be more related to BP levels than the urinary excretion of the two minerals alone (Dyer *et al*., *Hypertension,* 1994, 23, 729-36).

Epidemiological studies have indicated that populations or people accustomed to a high potassium intake have lower blood pressure levels (Langford, *Ann. Intern. Med.,* 1983, 98, 770-2; and Intersalt Cooperative Research Group, *Br. Med. J*., 1988, 297, 319-28) and have a very low occurrence of hypertension and related vascular diseases when compared with populations or people having a low potassium intake (*Frisancho* et al., *J*. *Chronic. Dis.,* 1984, 37, 515-9). A transmigrational study has also shown that BP was increased in people moving from an area of high-potassium intake to an area of low-potassium intake (Ward *et al., Hypertension,* 1980, 2 (Suppl. 1), I 43-54). A considerable number of randomised controlled trials on potassium supplementation, however, have produced inconclusive or conflicting results (Whelton *et al., J. Amer. Med. Assoc*., 1997, 277, 1624-32).

Some clinical studies have also indicated that the potassium-induced hypotensive effect increases with time (Cappuccio *et al., J. Hypertens*., 1991, 9, 465-73), becoming more pronounced after about six weeks of supplementation (Siani *et al., Br. Med. J.* (*Clin. Res. Ed.*), 1987, 294, 1453-6). Whelton *et al.* (*J. Amer. Med. Assoc*., 1997, 277, 1624-32) noted that despite the diversity of experimental designs that had been employed, nevertheless it seemed clear that the response to potassium supplementation was not directly related to dose level. In many of the studies reviewed by Whelton their duration was short, the number of subjects (predominantly hypertensive) was small, and the supplement of potassium was high, ranging from 60 to 200 mmol/day, an amount that is unlikely to be achieved by dietary means alone in most of the population.

The present invention provides methods and compositions for lowering BP by a modest increase in dietary potassium.

### SUMMARY OF THE INVENTION

The present invention provides methods of decreasing blood pressure in a mammal comprising administering to the mammal between about 10 mmol/day and about 35 mmol/day of a potassium salt.

The present invention also provides methods of treating or preventing hypertension in a mammal comprising administering to the mammal between about 10 mmol/day and about 35 mmol/day of a potassium salt.

The present invention also provides beverages supplemented with between about 10 mmol per daily serving and about 35 mmol per daily serving of a potassium salt.

The present invention also provides foodstuffs supplemented with between about 10 mmol per daily serving and about 35 mmol per daily serving of a potassium salt.

### BRIEF DESCRIPTION OF THE DRAWINGS

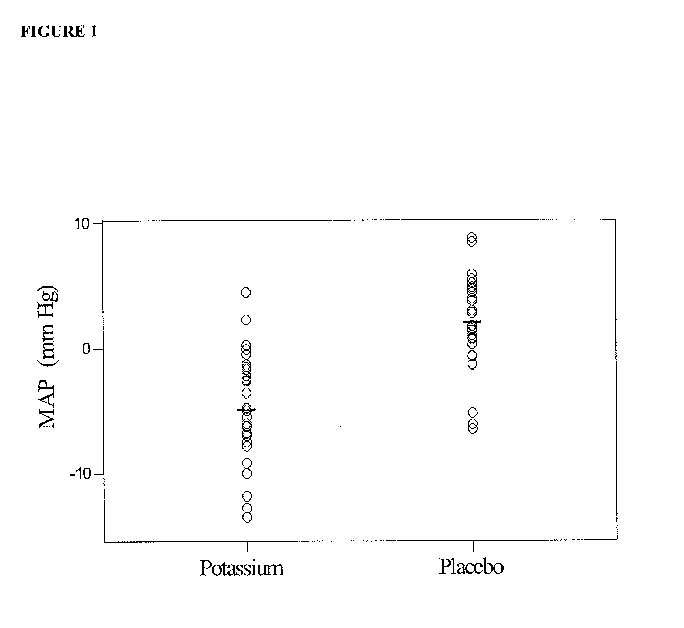

Figure 1 depicts a comparison of changes in mean arterial pressure (MAP) in the two groups receiving either the placebo or potassium chloride.

### DESCRIPTION OF PREFERRED EMBODIMENTS

The present invention provides methods of decreasing blood pressure or treating or preventing hypertension in a mammal comprising administering to the mammal between about 10 mmol/day and about 35 mmol/day of a potassium salt. The present invention also provides beverages and foodstuffs supplemented with a low dose a potassium salt. The mammal may be one in need of such decrease in blood pressure, or treatment or prevention of hypertension.

As used herein, "about" means ± 5% of the value it modifies. For example, "about" 10 mmol of potassium salt means a range of potassium salt from 9.5 to 10.5 mmol.

As used herein, "between" means the range of values set forth within and including two endpoints. For example, "between" about 10 mmol and 35 mmol of a potassium salt means an amount of potassium salt including 10 mmol and 35 mmol and all amounts of potassium salt greater than 10 mmol and less than 35 mmol.

As used herein, "daily serving" means that amount of supplement, beverage, or foodstuff to be administered per day. Such daily servings may be administered alternatively as a single serving, or two servings, or three servings in a day, such that the amount of potassium salt administered in a day equals the daily serving.

In some embodiments, methods of decreasing blood pressure in a mammal are provided. Between about 10 mmol and about 35 mmol of a potassium salt is administered to the mammal per day. Alternatively, between about 15 mmol and about 30 mmol, or between about 20 mmol and about 25 mmol, or between about 22 mmol and about 24 mmol of a potassium salt is administered to the mammal per day. These specific ranges are suitable for an average human, and for the vast majority of humans. The dosage of potassium salt administered may be varied, however, depending upon factors such as the type of mammal, pharmacodynamic characteristics, the mode and route of administration, age, health, and weight of the recipient mammal, nature and extent of symptoms, kind of concurrent treatment (e.g., concurrent treatment with antiinflammatories may require lowered potassium dosages), and frequency of treatment.

In some embodiments, mammals that have high blood pressure or are likely to have high blood pressure are identified. Thus, the methods of decreasing blood pressure in a mammal can comprise identifying such a mammal in need of a decrease in blood pressure. In some embodiments, the blood pressure of the mammal is decreased at least about 5% as measured by the mean arterial pressure of the mammal. Decreases in blood pressure can also be observed by measuring diastolic blood pressure (DBP) and systolic blood pressure (SBP).

In other embodiments of the invention, methods of treating or preventing hypertension in a mammal are provided. Between about 10 mmol and about 35 mmol of a potassium salt is administered to the mammal per day. Alternatively, between about 15 mmol and about 30 mmol, or between about 20 mmol and about 25 mmol, or between about 22 mmol and about 24 mmol of a potassium salt is administered to the mammal per day. These ranges are suitable for an average human. The dosage of potassium salt administered varies depending upon factors such as the type of mammal, pharmacodynamic characteristics, its mode and route of administration, age, health, and weight of the recipient mammal, nature and extent of symptoms, kind of concurrent treatment (e.g., concurrent treatment with antiinflammatories may require lowered potassium dosages), and frequency of treatment.

In some embodiments, mammals that have hypertension or are likely to have hypertension are identified. Thus, the methods of treating or preventing hypertension in a mammal can comprise identifying such a mammal having or suspected of having, or which are likely to have hypertension in the future. Factors that implicate hypertension or indicate that hypertension may be forthcoming include, but are not limited to, smoking, alcohol consumption, use of medications, physical activity, and stress. In some embodiments, the blood pressure of the mammal is decreased at least about 5% as measured by the mean arterial pressure of the mammal. Alternatively, hypertension in the mammal that otherwise would have resulted absent treatment is prevented.

Mammals include, but are not limited to, humans and non-human primates, livestock animals such as horses, cattle, sheep, and goats, and domestic animals, such as dogs, cats, mice, rats, guinea pigs, and rabbits.

Potassium salts include any salt of potassium that can be formulated so as to be administered to a mammal. In some embodiments, the potassium salt is a potassium halide such as potassium chloride or potassium iodide. Other embodiments include ionic compositions of potassium wherein the anion is an organic anion, such as but not limited to potassium tartrate, potassium citrate, potassium bicarbonate, potassium gluconate, potassium lactate, and the like.

In the methods of the present invention, the potassium salt can be administered in a number of ways. According to some methods of the invention, the potassium salt can be administered to a mammal at a site on said mammal's body by a route of administration selected from the group consisting of intramuscular, intranasal, intraperitoneal, subcutaneous, intradermal, topical, and oral. In some embodiments, the potassium is administered orally in the form of at least one tablet or a plurality of tablets. Such tablets may be swallowed whole, or may be dissolved in water or another beverage for drinking. Such tablets may be effervescent upon dissolving in liquid.

Tablets include, but are not limited to, slow-release tablets. The potassium salt can be combined or coadministered with a suitable carrier or vehicle. Suitable carriers are described in the most recent edition of *Remington's Pharmaceutical Sciences,* A. Osol, a standard reference text in this field, which is incorporated herein by reference in its entirety. Carriers include any pharmaceutically acceptable solid or liquid filler, diluent or encapsulating material. Carriers can contain pharmaceutically acceptable additives such as, for example, acidifying agents, alkalizing agents, antimicrobial preservatives, antioxidants, buffering agents, chelating agents, complexing agents, solubilizing agents, humectants, solvents, suspending and/or viscosity-increasing agents, tonicity agents, wetting agents or other biocompatible materials. Pharmaceutically acceptable carriers include, but are not limited to, sugars (e.g., lactose, glucose and sucrose), starches (e.g., corn starch and potato starch), cellulose and cellulose derivatives (e.g., sodium carboxymethyl cellulose, ethyl cellulose), excipients (e.g., cocoa butter and suppository waxes), oils (e.g., peanut oil, cottonseed oil, safflower oil, sesame oil, olive oil, corn oil and soybean oil), glycols and polyols (e.g., propylene glycol, glycerin, sorbitol, mannitol and polyethylene glycol), esters (e.g., ethyl oleate and ethyl laurate), agar, buffering agents and solutions (e.g., magnesium hydroxide, aluminum hydroxide, pyrogen free water, isotonic saline, Ringer's solution, ethyl alcohol and phosphate buffer solutions), and the like. Wetting agents, emulsifiers and lubricants (e.g., sodium lauryl sulfate and magnesium stearate), coloring agents, release agents, coating agents, sweetening, flavoring and perfuming agents, effervescing agents, preservatives, and antioxidants can also be present within the potassium salt formulations. Slow-release tablets (600 mg KCl) can be obtained from, for example, Alliance Pharmaceuticals Ltd. (Avonbridge House, Chippenham, Wiltshire SN15 2BB, UK).

In some embodiments, the potassium salt is administered concurrently within a reasonable time with an anti-hypertension compound. Such compounds are known in the art and include, but are not limited to, the following classes of compounds: diuretics, anxiety medication, alpha blockers, beta blockers, ACE inhibitors, calcium channel blockers, and vasodilators. Alternatively, the potassium salt is administered in the form of a supplemented beverage or foodstuff.

In other embodiments of the invention, beverages supplemented with potassium salt are provided. Between about 10 mmol and about 35 mmol of a potassium salt is administered to the mammal per day. Alternatively, between about 15 mmol and about 30 mmol, or between about 20 mmol and about 25 mmol, or between about 22 mmol and about 24 mmol of a potassium salt per day is included within the supplemented beverage. For example, if it were desired to supplement a mammal with about 22 mmol/day of potassium salt, the mammal can be orally administered 200 ml of a beverage supplemented with 22 mM potassium salt. The beverage can be any liquid including, but not limited to, fruit juices, vegetable juices, milk, water, carbonated beverages, sports beverages, health beverages, and the like. Fruit juices can contain orange juice, apple juice, grape juice, grapefruit juice, elixirs, syrups, or any combination thereof. Vegetable juices can contain tomato juice, carrot juice, or a combination thereof.

In other embodiments of the invention, foodstuffs supplemented with potassium are provided. The potassium can be in any form including a salt. Between about 10 mmol and about 35 mmol, or alternatively, between about 15 mmol and about 30 mmol, or between about 20 mmol and about 25 mmol, or between about 22 mmol and about 24 mmol of potassium is included within a daily serving of the foodstuff. For example, if it were desired to supplement a mammal with 24 mmol of potassium, a confection containing 8 mmol of potassium may be prescribed to be ingested three times per day. Foodstuffs which may be supplemented include, but are not limited to, confectionaries (e.g., chewing gum, savory snackfoods, candies, and the like), bakery goods, cereals, fruits, vegetables, and the like. Supplementation of foodstuffs may be performed by simple addition of potassium salt into the foodstuff, or by genetically modifying the foodstuff, or any other means which results in a foodstuff enriched in a potassium salt.

In order that the invention disclosed herein may be better understood, Examples are provided below. It should be understood that these Examples are for illustrative purposes only and are not to be construed as limiting the invention in any manner. In addition, the disclosures of each patent, patent application, and publication cited or described in this document are incorporated herein by reference in their entirety.

### EXAMPLES

### Example 1: Low-dose Potassium Trial Design

### Subjects

Seventy one subjects aged between 25 to 65 years among the academic and post-graduate research staff of King's College London took part in the following study. Fifty nine completed the study.

At the first visit, characteristics influencing BP (Beevers *et al*., ABC of hypertension, 4th Ed., London, BMJ Publishing Group, 2000) such as age, gender and ethnic origin were recorded. Participants were interviewed during the first appointment about their general health conditions and regarding the known lifestyle factors that may be involved in the determination of BP (*Id*.), such as smoking habit, alcohol consumption (units per week), use of medications and physical activity (see Table 1).

**Table 1**

| Classification of physical activity levels | |
|---|---|
| Class | Hours of physical activity/wk |
| 1 | ≤ 1 |
| 2 | between 1 and 2 |
| 3 | between 2 and 4 |
| 4 | ≥ 4 |

To obtain strongly motivated subjects, the volunteers were not attracted either with economic incentive or reimbursement for their participation. Written informed consent was obtained from each participant before taking part in the study, which was approved by the King's College London Research Ethics Committee for Research Involving Human Subjects.

### Exclusion Criteria

The main exclusion criteria were diseases or conditions that might involve collateral effects from potassium supplementation or interfere with its metabolism (Medline plus Drug information: Potassium Supplements (systemic), world wide web at nlm.nih.gov/medlineplus/
druginfo/potassiumsupplementssystemic202473.html). These were insulin dependent diabetes mellitus, non-insulin dependent diabetes mellitus, diabetes insipidus, cardiovascular diseases (including events of cardiac arrhythmia and peripheral arterial disease) or previous cardiovascular events, any kind of renal diseases, metabolic acidosis, current peptic ulcers, dysphagia, general digestive problems, gastric surgery, pregnancy and lactation. Other exclusion criteria were the use of antihypertensive drugs, and the use of drugs known to interfere with potassium metabolism.

To avoid confounding, the subjects who during the study period changed either their usual diet or other general lifestyle factors known to influence blood pressure were excluded from the study. Subjects taking other medications for which potassium supplementation was not contraindicated, or mineral and vitamin supplements, were allowed to participate in the study with the provision that they continued to use these products.

### Design of the Trial

A double-blind, placebo controlled trial on 59 volunteers, randomly assigned to receive 24 mmol/d of slow release potassium chloride (n=30) or a placebo (n=29) was conducted. Measures of BP, anthropometric characteristics and urine analysis for electrolytes were recorded during a one-week baseline period. Supplementation was for six weeks during which BP and changes in weight were assessed and a second 24-hour urine collection made. The primary outcome was the change in mean arterial pressure (MAP). Systolic (SBP) and diastolic (DBP) blood pressure were secondary outcomes.

The study took the form of a randomized double-blind, placebo-controlled trial with the aim of testing the effect of potassium supplementation on BP. The 71 subjects were randomly assigned to receive either a potassium supplement (n= 36) or a placebo (n= 35). The study consisted of a baseline period of one week during which blood pressure and body weight were measured at the beginning and end, and a 24-hour urine sample was collected to estimate electrolyte intake. During a six weeks intervention phase, BP and body weight were measured at three-week intervals, and a second 24-hour urine sample was collected during the final week. Potassium chloride was administered as one slow release tablet containing 8 mmol of potassium chloride (Slow-K Tablets 600 mg kindly supplied by Alliance Pharmaceuticals Ltd, Avonbridge House, Chippenham, Wiltshire SN15 2BB, UK) three times a day with meals. The placebo contained sorbitol and fructose.

The patients and the investigator were unaware of the type of supplement being given, other than that it was a nutrient. The placebo and potassium chloride pills were provided in identical containers. The adherence of the volunteers to the treatment was assessed at each visit by questioning, and by counting the pills returned.

### Blood Pressure, Heart Rate and Body Mass Index

Each BP measurement was performed following current recommendations (Beevers *et al*., ABC of hypertension, 4th Ed., London, BMJ Publishing Group, 2000). BP was assessed at the same time of day (within 30 minutes) for every appointment to avoid the influence of circadian variations in BP (*Id*.), at approximately the same heart rate, and by the same observer using the same instruments throughout the study for each measurement. The subjects were instructed not to smoke, eat or drink during the 30 minutes before each appointment. They were also given verbal and written notice to avoid exercising 30 minutes before the appointment, to come to the appointment with an empty bladder and to maintain strictly their usual habits on the day of the appointment (coffee, tea, meals consumed at the same time of the day). BP measurements were performed after the subjects had rested quietly for at least five minutes in the seated position. Two different cuffs (35 cm and 42 cm) were used depending on the arm circumference. At each visit at least three readings for systolic (SBP) and diastolic (DBP) BP and heart rate were taken at two-minute intervals in the left arm using a semi-automated device employing the oscillometric method (Omron M5-I, Omron Healthcare Europe B.V., Hoofddorp, The Netherlands). The first readings of each assessment were ignored to avoid results affected by the defense reaction (Beevers *et al*., ABC of hypertension, 4th Ed., London, BMJ Publishing Group, 2000) and the last two readings were averaged and recorded. The readings were thereafter confirmed after a two-minute interval with a Hawksley random zero sphygmomanometer (Hawksley and Sons, Lancing, Sussex, UK) because automatic devices may be unsuitable for some individuals for which there is no obvious reason (*Id*.).

SBP was measured as the point of appearance of the Korotkoff sounds (Phase I) and DBP was measured as the point of complete disappearance of the Korotkoff sounds (Phase V) according to the guidelines given by the British Hypertension Society (*Id*). All recordings from the semi-automated device agreed well with the random zero sphygmomanometer. Subjects with either SBP ≥ 140 mm Hg or DBP level ≥ 90 mm Hg were considered to be hypertensive.

Mean Arterial Pressure (MAP) was computed as MAP= (2 DBP + SBP)/3 (Vander *et al*., Human Physiology, The Mechanism of Body Function, 8th Ed., McGraw-Hill, New York, 2000, pp. 407-16 and 626-34, and Wilson *et al., Hypertension*, 1999, 34, 181-6).

### Urine Collection and Laboratory Analysis

The subjects were given instruction on providing the 24-hour urine collection for analysis for sodium, potassium and chloride. Urinary volumes were measured and an aliquot refrigerated for a few days before analysis. Urinary potassium and sodium were analyzed by flame photometry (Coming 410 Flame Photometer, Corning Science Products, Corning Limited, Halstead, Essex, CO9 2DX, England) and chloride content was assessed by potentiometric titration (926 Chloride Analyzer, Corning Medical and Scientific, Corning Limited, Halstead, Essex, CO9 2DX, England). For potassium and sodium analyses the linear regression of the standards and the interpolation of the data were performed using a Hewlett Packard 97 S I/0 Calculator (Hewlett HP Packard, 1000 N.E. Circle Blud, Corvallis, OR 97330, U.S.A). Sodium only was measured in the final sample.

An estimate of customary sodium intake was made by averaging the sodium content of the two urine collections. All the electrolyte data presented in Table 2 are derived from the first urine collection only.

**Table 2**

| Baseline characteristics of the participants in the study¹ | | |
|---|---|---|
| | Type of treatment | |
| | Potassium Chloride (n=30) | Placebo (n=29) |
| Age (y) | 44.5 ± 2.1² | 41.7 ± 2.2 |
| Women (%) | 53 | 35 |
| European (%) | 83 | 83 |
| Middle-Eastern (%) | 7 | 7 |
| South Asian (%) | 3 | 7 |
| East Asian (%) | 7 | 3 |
| BMI (kg/m²) | 26.06 ± 0.64 | 24.90± 0.82 |
| Physical activity (classes)³ | 2.10 ± 0.19 | 2.14 ± 0.18 |
| Alcohol consumption (units/wk) | 8.6 ± 1.5 | 10.3 ± 1.5 |
| Smokers (%) | 23 | 28 |
| Hypertensives (%) | 20 | 7 |
| Heart rate (pulses/min) | 66.6 ± 1.4 | 67.7 ± 2.0 |
| MAP (mm Hg) | 89.9 ± 2.1 | 85.6 ± 1.9 |
| SBP (mm Hg) | 118.2 ± 2.6 | 115.7 ± 2.4 |
| DBP (mm Hg) | 75.5 ± 2.1 | 70.5± 1.8 |
| Na (mmol/d) | 175.4 ± 13.0 | 155.1 ± 9.1 |
| Cl (mmol/d) | 170.6 ± 13.0 | 148.7 ± 8.9 |
| K (mmol/d) | 84.0 ± 4.5 | 84.0 ± 5.2 |
| Na-K ratio | 2.13 ± 0.13 | 2.01 ± 0.17 |

| | | |
|---|---|---|
| ¹There were no significant differences at baseline between the two groups (Two-Sample *t* test or Chi-square test) | | |
| ² mean ± SEM | | |
| ³ Calculated as described in Table 1 | | |

### Outcomes

The mean difference in MAP change between the potassium supplemented and the placebo group was chosen as the primary outcome, while mean changes in DBP and SBP were chosen as secondary outcomes. This choice was made to evaluate the overall change in blood pressure because the MAP summarizes both SBP and DBP with a single value. Moreover, SBP and DBP may change within each individual with different patterns and sign, making it difficult to determine the direction and importance of changes in blood pressure.

### Statistical Analysis

The baseline blood pressure used for the statistical analysis was the average of the measurements taken during the first week period. For Interval level variables (e.g. BP), differences between groups were tested using the Two-Sample t test while differences within each group were tested with the Paired t test. For Ordinal level variables (e.g. compliance) these tests were performed using the Mann-Whitney Test and the Wilcoxon Signed Rank Test for comparisons between and within the two groups respectively. The Chi-Squared Test was used to assess the significance of differences between the groups for Nominal level variables (e.g. gender).

Correlations between the different variables and both BP and changes in BP, were initially tested using a Stepwise Regression analysis. Thereafter a regression analysis was performed based on the variables present in the final Stepwise model. Data are presented as means ± standard error of the mean (SEM) and 95% confidence intervals (CI). A P value of ≤ 0.05 was considered to be statistically significant. All calculations were performed with the Minitab statistical package (Minitab release 13.1, Minitab Inc., 3081 Enterprise Drive, State College, PA 16801-3008, U.S.A.)

### Example 2: Low-dose Potassium Trial Results

### Subject Details

Seventy one subjects were enrolled in the study, of whom 59 completed. Three subjects in both groups quit the study during the baseline period. In the active group, three volunteers were excluded because of changes in lifestyle, while in the placebo group, two retired for personal reasons, and one was excluded because of change in medication.

Baseline characteristics are shown in Table 2. There were no significant differences between the two groups with regard to age, body mass index (BMI), urinary electrolyte excretion, sodium-potassium ratio, blood pressure levels, heart rate, degree of physical activity and smoking habit. The number of subjects being of non-European ethnic origin and the number of hypertensives was small and so did not justify a separate statistical analysis for these subgroups. The potassium group contained more females than did the placebo group (53 % compared with 35 %) but the difference was not statistically significant and is not likely to have influenced the results of this study since no significant correlation was found between mean changes in BP and gender as shown by the stepwise regression analysis.

Therefore, the effect on BP of potassium supplementation could be evaluated by comparing the mean changes in blood pressure level in the two entire intervention groups.

### Compliance & Side effects

The overall compliance to the treatment was very satisfactory being 89 % (± 2.5, SEM) for the potassium group and 83 % (± 3.1, SEM) for the controls. The difference was not statistically significant.

One person receiving potassium claimed that he experienced an increase in appetite, although neither his weight nor his physical activity changed during the study. Two subjects in the placebo group reported side effects. One experienced occasional nausea associated with the consumption of the pills, while another subject reported transitory polyuria during the first week of treatment. Both volunteered to remain in the study, and their unexpected symptoms rapidly disappeared.

### Blood pressure

Among the baseline characteristics, all measures of BP (MAP, SBP and DBP) were positively related only to BMI (P < 0.001, P < 0.003, P < 0.001 respectively) and age (P < 0.002, P < 0.001, P < 0.006 respectively).

During treatment BP values changed significantly in both groups (Table 3). In the potassium group, at the end of the six-week intervention period MAP, SBP and DBP had decreased significantly when compared with the baseline values. This decrease occurred gradually. A significant decrease in MAP was found at the end of the first 3-week intervention period, driven mainly by a significant decrease in DBP while in the same period SBP was not significantly reduced. In contrast, during the second three-week period SBP decreased significantly contributing to a further fall in MAP.

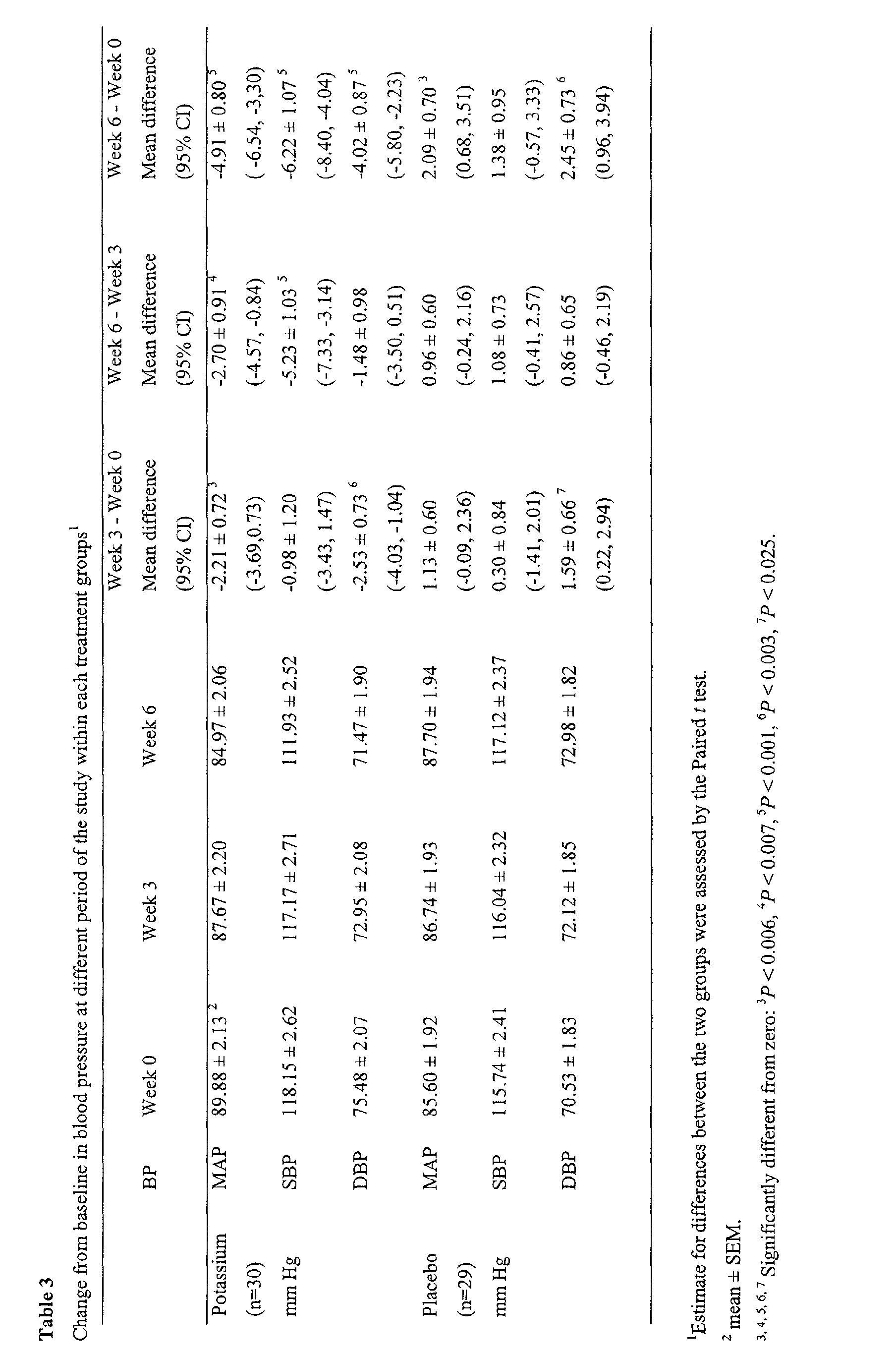

At the end of the study, in the placebo group MAP had increased significantly due in great part to a significant change in DBP noted, again, after the first 3 weeks of treatment. Comparisons between the mean changes in BP measurements of the two experimental groups demonstrate that the group treated with potassium produced a marked and significant decrease in SBP and DBP, and thus in MAP (as shown in Table 4) when compared to the group given the placebo. The decrease in BP occurred continuously during the length of the study, following a pattern similar to that described above for the changes in BP measurements from baseline values, but being more accentuated by the concomitant negative changes of BP values in the active group and positive changes in BP in the placebo group. A comparison of changes in MAP in the two groups is shown graphically in Figure 1.

Regression analysis revealed that the changes in MAP and DBP induced by potassium supplementation were positively related to the baseline urinary Na-K ratio (P < 0.035 and P < 0.022 respectively), while the change in DBP was positively related to the baseline SBP (P < 0.017) and negatively related to baseline DBP (P < 0.002).

**TABLE 4**

| Comparisons of mean changes in blood pressure levels between the potassium and the placebo group during the different periods of the study¹. | | | | |
|---|---|---|---|---|
| | | Type of treatment | | |
| Period | Blood pressure | Potassium (n=30) | Placebo (n=29) | Potassium - placebo Mean Difference (95% CI) |
| Week 0 to 3 | MAP (mm Hg) | -2.21 ± 0.72 ² | 1.13 ± 0.60 | -3.34 (-5.22, - 1.47) ³ |
| | SBP (mm Hg) | -0.98 ± 1.20 | 0.30 ± 0.83 | -1.28 (-4.21, 1.65) |
| | DBP (mm Hg) | -2.53 ± 0.73 | 1.58 ± 0.66 | -4.11(-6.09, -2.13) ⁴ |
| Week 3 to 6 | MAP (mm Hg) | -2.70 ± 0.91 | 0.96 ± 0.59 | -3.66(-5.84, - 1.49) ³ |
| | SBP (mm Hg) | -5.23 ± 1.00 | 1.08 ± 0.73 | -6.32 (-8.84, -3.79) ⁴ |
| | DBP (mm Hg) | -1.48 ± 0.97 | 0.87 ± 0.65 | -2.35 (- 4.70, -0.00) ⁵ |
| Week 0 to 6 | MAP (mm Hg) | -4.91 ± 0.79 | 2.09 ± 0.69 | -7.01 (-9.12, -4.89) ⁴ |
| | SBP (mm Hg) | -6.22 ± 1.10 | 1.38 ± 0.95 | -7.60 (-10.46, -4.73) ⁴ |
| | DBP (mm Hg) | -4.02 ± 0.87 | 2.45 ± 0.73 | -6.46 (-8.74, -4.19) ⁴ |

| | | | | |
|---|---|---|---|---|
| ¹Estimate for differences between the two groups (potassium - placebo) were assessed by the Two Sample *t* test | | | | |
| ² mean ± SEM | | | | |
| ^{3,4,5} Significantly different from zero: ³*P* < 0.002, ⁴*P* < 0.001, ⁵*P* = 0.050 | | | | |

### BMI & Heart Rate

In neither of the groups did BMI or heart rate change significantly during the course of the investigation, nor was there a significant difference between the two groups (Tables 5 and 6).

**Table 5**

| Comparison of the BMI of the participants in the study before and after the six-week treatment period¹ | | | |
|---|---|---|---|
| Treatment | Week 0 | Week 6 | Week 6 - Week 0 |
| | | | Mean difference |
| | | | (95% CI) |
| Potassium | 26.06 ± 0.64² | 26.09 ± 0.65 | 0.03 ± 0.07 |
| (n = 30) | | | (-0.12, 0.18) |
| Placebo | 24.90 ± 0.82 | 25.02 ± 0.86 | 0.12 ± 0.75 |
| (n = 29) | | | (-0.03, 0.28) |

| | | | |
|---|---|---|---|
| ¹There were no significant differences between and within the two groups after the six-week intervention period (Two-Sample *t* test or Paired *t* test) . | | | |
| ² mean ± SEM | | | |

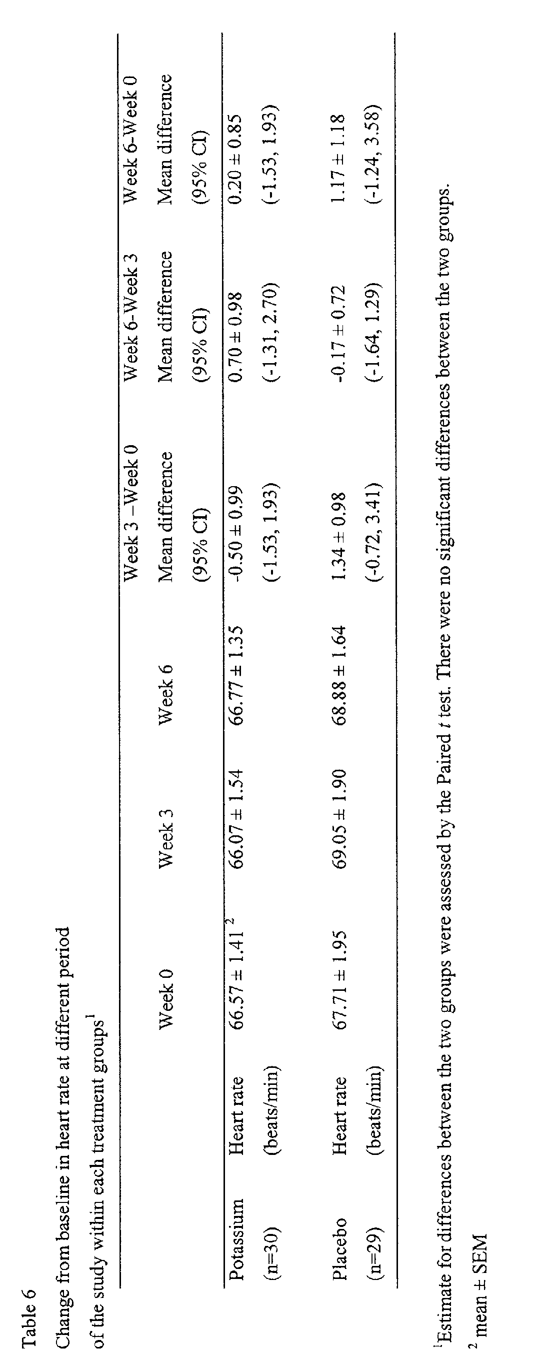

The effects of a modest potassium supplement on BP were studied in apparently healthy volunteers using a randomized double-blind, placebo-controlled design. Compliance in the active group was high, indicating that the subjects, on average, consumed 22 mol/day of potassium chloride.

A small but significant rise in MAP was evident in all but 6 of the placebo group, including hypotensive, normotensive, and hypertensive subjects (see Figure 1). This surprising observation is attributed to a change in ambient temperature between the one-week baseline period, when baseline measurements were recorded, and the ensuing six-week intervention period. The influence of temperature on BP is well documented (Jansen *et al., J. Hum. Hypertens*., 2001, 15, 113-7; and Giaconi *et al., Hypertension*, 1989, 14, 22-7). Between the two periods there was a notable drop in temperature of around 12C (from around 30C to 18C). It is likely, therefore, that during the first week of the study the unusually high temperature had a hypotensive effect resulting from two different heat-induced mechanisms: a decrease in cardiac output arising from depletion of the plasma volume secondary to sweating and a decrease in peripheral resistance to blood flow due to vasodilation (Vander *et al*., Human Physiology, The Mechanism of Body Function, 8^{th} Ed., McGraw-Hill, New York, 2000, pp. 407-16 and 626-34). All subjects taking part in the study, however, were subjected to the same environmental conditions; the influence of the change in temperature would thus apply equally to the active group.

MAP was significantly reduced following dietary potassium supplementation, the decrease being progressive over the six-week period of observation, with an early decrease in DBP in the first three-week period followed by a decrease in SBP in the latter half of the study. It has previously been suggested that at least six weeks of supplementation might be needed to reveal a significant change in BP (*Matlou* et al., J. Hypertens., *1986, 4, 61-4; and* Kawano *et al., Am. J. Hypertens*., 1998, 11, 141-6). In a trial lasting 15 weeks, it was noted that the greater rate of decrease in BP occurred between the third and sixth week of supplementation, while during the subsequent nine weeks, the rate of decrease was extremely small (*Siani* et al., Br. Med. J. (Clin. Res. Ed.), *1987, 294, 1453-6*). These observations have certain implications, since most of the clinical trials on which meta-analyses have been based (*Whelton* et al., J. Amer. Med. Assoc., *1997, 277, 1624-32; and Cappuccio* et al., J. Hypertens., *1991, 9, 465-73)* have lasted less than six weeks.

The reduction in MAP described herein (7.01 mm Hg) was comparable to that achieved by single drug-therapy for hypertension (Materson *et al., N. Engl. J. Med.,* 1993, 328, 914-21; *erratum, N. Engl. J. Med.,* 1994, 330, 1689), and was substantial when compared with the mean decrease calculated from 33 randomized trials (*Whelton* et al., J. Amer. Med. Assoc., *1997, 277, 1624-32*). MAP fell by 3.4 mmHg in the studies of at least six weeks duration, in which the potassium supplement ranged from 60 to 120 mmol/d. In a recent trial, 18 hypertensive patients received a daily supplement of 48 mmol of potassium chloride (*Siani* et al., Br. Med. J. (Clin. Res. Ed.), *1987, 294, 1453-6*) and resulted in lower BP. The apparently greater efficacy of low-dose supplementation may have an explanation in the nature of the anion ingested with potassium. It has been claimed that the pressor effect of sodium is exerted only when sodium is consumed as the chloride salt (Kotchen *et al., Am. J. Clin. Nutr.,* 1997, *65* (2 Suppl), 708S-11S; Shore *et al., J. Hypertens*., 1988, 6, 613-617; and Boegehold *et al., Hypertension,* 1991, 17 (1 Suppl), I 158-61). A high intake of chloride may therefore antagonize the effect of potassium in high-dose supplementation.

When compared with the marginal benefits arising from a dietary-salt restriction, the effects of a modest potassium supplement are surprisingly encouraging. The results of the Intersalt Study (Intersalt Cooperative Research Group, *Br. Med. J.,* 1988, 297, 319-28) predict that a decrease in sodium intake of 100 mmol/d (5.8 g salt) would be associated with a decrease in BP of 3.14/ 0.14 mm Hg, and a recent meta analysis of randomized clinical trials (*Graudal* et al., J. Amer. Med. Assoc., *1998, 279, 1383-91)* found that a reduction in dietary sodium of 100 mmol/d promoted a small but significant decrease in BP of 0.8/0.2 and 3.6/1.6 mm Hg in normotensive and hypertensive subjects respectively.

Sodium and potassium have contrary effects on BP but the relationship is more subtle. Evidence from epidemiological studies and clinical trials (Dyer *et al., Hypertension,* 1994, 23, 729-36; and Geleijnse *et al., Brit. Med. J.,* 1990, 300, 899-902) has indicated that the sodium-potassium ratio is positively correlated with BP, and that the nature of this association appears to be stronger than that for urinary potassium and sodium alone. The recent DASH salt study (Sacks *et al., N. Engl. J. Med.,* 2001, 344, 3-10) comprised two dietary groups, a typical American diet and the DASH diet proposed for the reduction of cardiovascular and cerebrovascular diseases in the U.S. population, but adjusted to provide the same amount of sodium. At baseline, the difference in BP between the groups was 5.9/2.9 mm Hg, almost three times as great as the fall in BP induced by a reduction in sodium intake of 50 mmol/d. It is noteworthy that the DASH diet provided twice as much potassium. When, however, the sodium content of the diets was restricted by a further 50 mmol/d, the effect of potassium was attenuated, the difference between the dietary groups falling to 2.2/1.0 mm Hg. Clearly potassium supplementation is most beneficial in subjects consuming a high salt diet (Whelton *et al., J. Amer. Med. Assoc*., 1997, 277, 1624-32; and Kawano *et al., Am. J. Hypertens*., 1998, 11, 141-6), as observed in our subjects, who had a mean salt intake of 9.5 g/d. Conversely, salt sensitivity, defined as an increase in MAP resulting from salt loading (Morris *et al., Hypertension*, 1999, 33, 18-23), is dose-dependently suppressed when dietary potassium is increased within the normal range of intake.

The recommended intake of potassium in the UK (Department of Health, Report on health and social subjects 41, Dietary Reference Values for Food Energy and Nutrients for the United Kingdom, Committee on Medical Aspects of Food Policy, HMSO, London, 1991, 152-60) is 90 mmol/d, some 20 mmol above the average consumption (Gregory *et al*., The Dietary and Nutritional Survey of British Adults, HMSO, London, 1990, 154-8). A general and chronic low-grade potassium deficiency, therefore, affects a large part of the population, the occurrence being particularly striking in the elderly (*Bates* et al., Br. J. Nutr., *1999, 82, 7-15)* and among those belonging to the low social-economic classes (Smith *et al., Proc. Nutr. Soc.,* 1997, 56, 75-90). As a means of reducing the incidence of cardiovascular and cerebrovascular disease, diet modification involving an increased consumption of fresh fruits and vegetables may be unrealistic because of their relatively high cost. Eight of our subjects (14 %), none of whom was aware of any abnormality in BP, were found to be hypertensive during the course of the study, a finding that highlights the importance of ensuring, *inter alia*, an adequate intake of potassium. The present invention, diet supplementation with potassium or fortification of processed foods, offers an inexpensive and safe preventive measure to confront a major public health problem.

In sum, after six weeks of supplementation, MAP was reduced by 7.01 (95 % CI: -9.12, - 4.89; P < 0.001) mm Hg, SBP was reduced by 7.60 (95 % CI: -10.46, - 4.73; P < 0.001) mm Hg and DBP was reduced by 6.46 (95 % CI: -8.74, -4.19; P < 0.001) mm Hg. The reduction in MAP was positively associated with the baseline urinary sodium-potassium ratio (P < 0.034). Thus, a low daily dietary supplement of potassium, equivalent to the content of 5 portions of fresh fruits and vegetables, induced a substantial reduction in MAP, similar in effect to single drug-therapy for hypertension.

### Example 3: Low-Dose Potassium Supplemented Beverages

Low-dose potassium supplemented beverages are formulated such that a daily serving provides from about 10 to about 35 mmol of additional potassium. For example, potassium chloride is added to orange juice such that a six ounce serving contains an additional 8 mmol of potassium; thus three six-ounce servings provides a supplement of 24 mmol of potassium. Information provided by packaging, labeling, or the like may be provided indicating that three servings per day are recommended. Alternatively, potassium chloride is added to orange juice such that a six ounce serving contains an additional 12 mmol of potassium; thus two six-ounce servings provides a supplement of 24 mmol of potassium. Information provided by packaging, labeling, or the like may be provided indicating that two servings per day are recommended.

By way of further example, potassium bicarbonate is added to orange juice such that a six ounce serving contains an additional 8 mmol of potassium; thus three six-ounce servings provides a supplement of 24 mmol of potassium. Information provided by packaging, labeling, or the like may be provided indicating that three servings per day are recommended. Alternatively, potassium chloride is added to orange juice such that a six ounce serving contains an additional 12 mmol of potassium; thus two six-ounce servings provides a supplement of 24 mmol of potassium. Information provided by packaging, labeling, or the like may be provided indicating that two servings per day are recommended.

By way of further example, potassium citrate is added to orange juice such that a six ounce serving contains an additional 8 mmol of potassium; thus three six-ounce servings provides a supplement of 24 mmol of potassium. Information provided by packaging, labeling, or the like may be provided indicating that three servings per day are recommended. Alternatively, potassium chloride is added to orange juice such that a six ounce serving contains an additional 12 mmol of potassium; thus two six-ounce servings provides a supplement of 24 mmol of potassium. Information provided by packaging, labeling, or the like may be provided indicating that two servings per day are recommended.

The amount of potassium in a serving as described above may be altered such that the daily serving amount is between from about 10 to about 35 mmol, or preferably from about 15 to about 30 mmol, more preferably from about 20 to about 25 mmol, or more preferably from about 22 to about 24 mmol.

Likewise, the individual serving size may likewise be altered, so long as the total daily serving falls within the above-described ranges. For example, individual serving sizes of about one teaspoon or one tablespoon (e.g., in an elixir, syrup, or the like) which provides half or one third of the daily serving, to be taken twice or three times per day, respectively, is contemplated. Further, serving sizes of between about 1 oz. and about 16 oz. are within the scope of the invention, provided that two or three individual servings provide a daily serving total of supplemental potassium in the ranges specified above.

As an additional example, the supplemental potassium is added to a bottled water product as described above for orange juice. As an additional example, the supplemental potassium is added instead to a carbonated beverage. As an additional example, the supplemental potassium is added instead to a sports beverage. As an additional example, the supplemental potassium is added instead to a health beverage.

Various modifications of the invention, in addition to those described herein, will be apparent to those skilled in the art from the foregoing description. Such modifications are also intended to fall within the scope of the appended claims. Each reference cited in the present application is incorporated herein by reference in its entirety.

## Claims

1. A method of decreasing blood pressure in a mammal comprising administering to the mammal between about 10 mmol/day and about 35 mmol/day of a potassium salt.

2. A method of treating or preventing hypertension in a mammal comprising administering to the mammal between about 10 mmol/day and about 35 mmol/day of a potassium salt.

3. The method of claim 1 or claim 2 wherein between about 15 mmol/day and about 30 mmol/day of a potassium salt is administered to the mammal.

4. The method of claim 1 or claim 2 wherein between about 20 mmol/day and about 25 mmol/day of a potassium salt is administered to the mammal.

5. The method of claim 1 or claim 2 wherein between about 22 mmol/day and about 24 mmol/day of a potassium salt is administered to the mammal.

6. The method of any one of claims 1 to 5 wherein the potassium salt is selected from the group consisting of potassium chloride, potassium citrate, potassium bicarbonate, potassium tartrate, potassium gluconate, and potassium lactate.

7. The method of claim 6 wherein the potassium salt is potassium chloride.

8. The method of claim 6 wherein the potassium salt is potassium bicarbonate.

9. The method of any one of claims 1 to 8 wherein the potassium salt is administered in the form of at least one tablet.

10. The method of any one of claims 1 to 8 wherein the potassium salt is administered in the form of a plurality of tablets administered at different times in a day.

11. The method of any one of claims 1 to 8 wherein the potassium salt is administered in the form of a supplemented beverage or a supplemented foodstuff.

12. The method of any one of claims 1 to 11 wherein the mammal is a human.

13. The method of any one of claims 1 to 12 wherein the blood pressure of the mammal is decreased at least about 5% as measured by the mean arterial pressure of the mammal.

14. The method of any one of claims 1 to 13 wherein the mammal is also coadministered an anti-hypertension compound.

15. The method of claim 14 wherein the anti-hypertension compound is a member selected from the group consisting of diuretics, anxiety medication, alpha blockers, beta blockers, ACE inhibitors, calcium channel blockers, and vasodilators.

16. A beverage supplemented with a potassium salt such that a daily serving provides between about 10 mmol and about 35 mmol of the potassium salt.

17. The beverage of claim 16 which provides a daily serving of potassium salt between about 15 mmol and about 30 mmol.

18. The beverage of claim 16 which provides a daily serving of potassium salt between about 20 mmol and about 25 mmol.

19. The beverage of claim 16 which provides a daily serving of potassium salt between about 22 mmol and about 24 mmol.

20. The beverage of any one of claims 16 to 19 which is a supplemented beverage selected from the group consisting of supplemented water, fruit juice, vegetable juice, carbonated beverage, sports beverage, health beverage, elixir, and syrup.

21. The beverage of claim 20 which comprises at least one member of the group consisting of orange juice, apple juice, grape juice, grapefruit juice, tomato juice, carrot juice, and any combination thereof.

22. The beverage of any of claims 16-21, wherein the potassium salt is is selected from the group consisting of potassium chloride, potassium citrate, potassium bicarbonate, potassium tartrate, potassium gluconate, and potassium lactate.

23. A foodstuff supplemented with a potassium salt such that a daily serving provides between about 10 mmol and about 35 mmol of the potassium salt.

24. The foodstuff of claim 23 which provides a daily serving of potassium salt between about 15 mmol and about 30 mmol.

25. The foodstuff of claim 23 which provides a daily serving of potassium salt between about 20 mmol and about 25 mmol.

26. The foodstuff of claim 23 which provides a daily serving of potassium salt between about 22 mmol and about 24 mmol.

27. The foodstuff of any of claims 23-26 wherein the potassiums salt is selected from the group consisting of potassium chloride, potassium citrate, potassium bicarbonate, potassium tartrate, potassium gluconate, and potassium lactate.

28. The beverage of claim 22 wherein the potassium salt is potassium chloride or potassium bicarbonate.

29. The foodstuff of claim 27 wherein the potassium salt is potassium chloride or potassium bicarbonate.

30. Use of a beverage of claim 22 or claim 28 for the treatment or prevention of hypertension.

31. Use of a foodstuff of claim 27 or claim 29 for the treatment or prevention of hypertension.
